Europäisches Patentamt

European Patent Office

Office européen des brevets

(11) Numéro de publication: **0 299 827 B1**

## (12) FASCICULE DE BREVET EUROPEEN

(45) Date de publication de fascicule du brevet: **23.12.92**  (51) Int. Cl.⁵: $A61B\ 5/02$, $A61B\ 8/04$

(21) Numéro de dépôt: **88401652.8**

(22) Date de dépôt: **28.06.88**

(54) **Dispositif de mesure de la pression sanguine dans une artère superficielle.**

(30) Priorité: **03.07.87 FR 8709448**

(43) Date de publication de la demande:
**18.01.89 Bulletin 89/03**

(45) Mention de la délivrance du brevet:
**23.12.92 Bulletin 92/52**

(84) Etats contractants désignés:
**AT BE CH DE ES GB IT LI NL SE**

(56) Documents cités:
FR-A- 2 045 853        FR-A- 2 481 918
US-A- 2 799 270        US-A- 3 099 262
US-A- 4 127 114        US-A- 4 320 767

(73) Titulaire: **Boutin, Gérard**
**4, Avenue Ziem**
**F-06800 Cagnes sur Mer(FR)**

(72) Inventeur: **Boutin, Gérard**
**4, Avenue Ziem**
**F-06800 Cagnes sur Mer(FR)**

(74) Mandataire: **Boutin, Antoine**
**Cabinet Tony-Durand, 77, Rue Boissière**
**F-75116 Paris(FR)**

# Description

La présente invention est relative à une sonde de mesure de la pression du sang dans une artère superficielle du corps humain, située sous et au voisinage de la peau d'un patient, et qui soit palpable par le doigt.

On connaît déjà, notamment par le brevet français 2.481.918, dont l'un des copropriétaires est le présent demandeur, des sondes de mesure de la pression sanguine dans les artères radiales du corps humain, en particulier mais non exclusivement pour la détermination de ce qu'il est convenu d'appeler, en médecine traditionnelle chinoise, le "pouls chinois", consistant à déterminer la pression artérielle en plusieurs endroits de l'artère, dont les emplacements correspondent à des repères anatomiques précis. De la comparaison des perceptions ressenties en ces différents points, le praticien peut, avec l'expérience et le savoir particulier correspondant à ce genre de médecine, formuler un diagnostic qui, joint à l'analyse d'autres symptômes éventuels, permet de définir la cause d'une affection et la thérapeutique de soin la plus appropriée. Selon ce brevet antérieur, on prévoit un appareil de mesure qui provoque en chaque point ainsi localisé, une cessation temporaire de l'écoulement du flux sanguin par pression et écrasement limité de l'artère correspondante, cet appareil comportant par ailleurs une sonde DOPPLER montée parallèlement pour fournir une onde ultra-sonore qui se réfléchit sur les éléments figurés du sang en circulation et dont le niveau est proportionnel à la vitesse du sang dons l'artère, l'extinction du son repérant l'arrêt de la circulation sanguine, ledit appareil étant agencé pour déduire de ces mesures, les valeurs de paramètres caractéristiques du pouls ainsi relevé, par l'élément de compression qui permet la mesure à l'extinction de l'onde DOPPLER.

Le brevet US 4.127.114 décrit pour sa part un autre appareil de mesure de la pression artérielle qui comporte également une sonde Doppler. Toutefois dans cet appareil la sonde Doppler est logée à l'intérieur d'une enceinte fermée dont l'extrémité est constituée par une membrane flexible.

Ceci permet de provoquer une pression maximum à l'intérieur de cette enceinte en appliquant la sonde correspondante sur la peau du patient. Cependant il est prévu un trou calibré d'échappement de l'air afin d'assurer ensuite une décompression progressive. Au passage de l'ondée systolique, la sonde Doppler mesure alors la pression maximum et, lors de la disparition de l'ondée systolique, cette même sonde détermine la pression minimum.

Un tel appareil présente pour inconvénient que la mesure de la pression est assurée avec interposition de la couche d'air comprimé dans l'enceinte déformable contenant la sonde Doppler. Il ne s'agit donc pas d'une mesure directe. Par ailleurs, l'emploi de cet appareil est peu aisé en pratique. Enfin il s'agit d'un appareil relativement complexe et coûteux compte tenu de l'agencement de celui-ci.

C'est pourquoi la présente invention a pour but de réaliser un dispositif de mesure de la pression sanguine qui soit aussi simple que possible et peu coûteux et dont l'emploi est particulièrement aisé. A cet effet, l'invention concerne un dispositif de mesure qui, tout en reprenant les principes généraux présidant à la conception de l'appareil décrit dans le brevet précité FR 2.481.918, y apporte des avantages structurels et fonctionnels particuliers, qui permettent notamment une utilisation de l'appareil plus sûre, plus pratique et qui surtout n'exige qu'un matériel peu coûteux et d'un usage très aisé.

L'invention vise notamment la réalisation d'un appareil capable de mesurer la pression artérielle en n'importe quel endroit d'une artère superficielle susceptible d'être palpée manuellement sous la peau et les tissus sous-cutanés, la mesure effectuée pouvant être répétitive et effectuée en tout point souhaité de l'artère, tant que celle-ci reste superficielle, afin de permettre un contrôle précis des éventuelles variations de la pression sanguine mesurée. Elle s'applique en outre plus particulièrement bien, quoique non exclusivement, à la mesure du pouls chinois sur les artères radiales d'un patient, notamment au niveau du poignet de celui-ci en divers repères exactement définis par la connaissance de cette technique, ces repères se répartissant dans ce cas sur une surface très réduite, de l'ordre du centimètre carré, ce qui exige de l'appareil de mesure une approche ponctuelle relativement très fine, pour différencier chaque mesure de la suivante.

A cet effet, le dispositif considéré, comportant une tête de mesure, propre à venir appuyer sur la peau du patient pour comprimer l'artère superficielle en provoquant, pour une pression donnée, l'arrêt de la circulation sanguine dans cette artère et, montée dans cette tête elle-même et non plus séparément, une sonde DOPPLER, fournissant une onde ultra-sonore, réfléchie par le sang en circulation et dont l'extinction détermine le moment où se produit l'arrêt de cette circulation, se caractérise en ce que la tête de mesure est solidaire d'un piston monté coulissant dans un corps fixe, délimitant une chambre étanche remplie d'air comprimé dont le volume est variable selon la position de la tête par rapport au corps, la pression de l'air contenu dans la chambre étant mesurée par un ensemble de contrôle associé donnant, après étalonnage, la valeur de la pression artérielle à tout instant, et en particulier à l'arrêt de la circulation sanguine dans l'artère, détecté par la sonde DOPPLER.

Dans un mode de réalisation particulier de l'invention, le piston solidaire de la tête de mesure est monté coulissant dans le corps sous l'effet d'une pression d'air comprimé croissante, fourni par une source de pression raccordée à la chambre à l'opposé de la tête, la pression de l'air dans la chambre étant mesurée en permanence par un manomètre de lecture. Selon le cas, la source de pression est constituée par un compresseur ou plus simplement par une pompe à main, gonflable, munie d'une valve de non retour et d'une soupape d'échappement.

Selon une autre caractéristique particulière de l'invention, la tête de mesure est constituée par un élément cylindrique allongé, présentant à son extrêmité opposée à celle venant comprimer l'artère superficielle, une partie filetée solidaire du piston coulissant. Avantageusement, la tête est constituée par un bloc en résine durcissable dans lequel est noyé la sonde DOPPLER.

Selon encore une disposition en elle-même connue mais avantageusement mise en oeuvre dans l'appareil selon l'invention, la sonde DOPPLER comporte deux cristaux transducteurs, respectivement émetteur et récepteur réunis l'un à un oscillateur et l'autre à un détecteur, ces cristaux étant immobilisés dans la tête de manière à présenter une inclinaison sur l'axe de l'élément cylindrique égale ou voisine de 45°.

De préférence, le manomètre de lecture fournissant la valeur instantanée de la pression dans la chambre est étalonné en centimètres de mercure. De préférence également et selon une autre caractéristique de l'invention, le corps fixe comporte un capot extérieur muni d'un bord rentrant librement traversé par l'élément cylindrique de la tête de mesure et formant butée d'arrêt pour le piston coulissant.

Dans une autre variante de réalisation de l'invention, le corps fixe enferme un volume d'air déterminé, dont la pression augmente consécutivement à l'enfoncement du piston coulissant solidaire de la tête de mesure à l'intérieur de la chambre étanche, la valeur de la pression étant lue par un capteur associé à un affichage directement porté par le corps.

Dans l'une ou l'autre des deux variantes de réalisation plus spécialement prévues, l'ensemble de contrôle dont la réalisation particulière ne concerne pas directement l'invention, est muni de moyens de lecture de la fréquence des ondes ultra-sonores fournies par la sonde DOPPLER et, le cas échéant, d'un enregistreur graphique des variations de cette fréquence. Avantageusement, l'ensemble de contrôle comporte également un ensemble logique, associé à un écran à cristaux liquides ou autre, comprenant une mémoire pour conserver les enregistrements des mesures et fournir une indication résultant de la comparaison de ces enregistrements.

D'autres caractéristiques d'un dispositif de mesure de la pression sanguine dans une artère superficielle, établi conformément à l'invention, apparaîtront encore à travers la description qui suit de plusieurs exemples de réalisation, donnés ci-après à titre indicatif et non limitatif, en référence aux dessins annexés sur lesquels :

- La Figure 1 représente une vue schématique en perspective et en coupe partielle d'un fragment de la peau et d'une artère superficielle dont la pression est à mesurer au moyen d'un dispositif selon l'invention.
- La Figure 2 est une vue de détail à plus grande échelle de l'extrêmité de la tête de mesure du dispositif considéré.
- La Figure 3 est une vue en coupe schématique du dispositif de l'invention.
- La Figure 4 est une vue en perspective et à plus faible échelle, d'un ensemble utilisant le dispositif selon l'invention, pour la mesure de la pression sanguine dans une artère superficielle d'un patient.

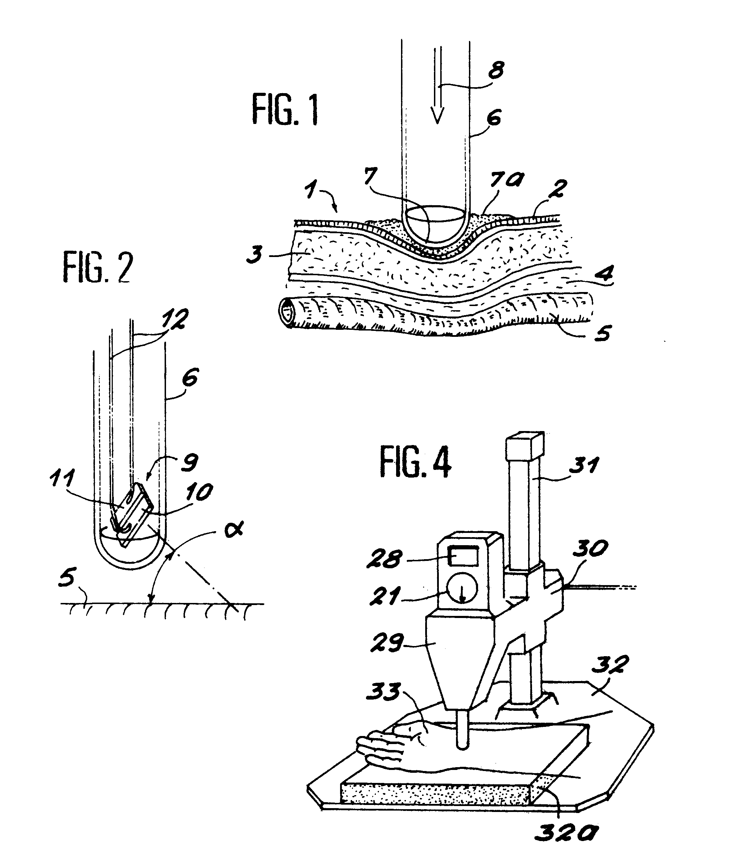

Sur la Figure 1, la référence 1 désigne de façon générale, un fragment 1 de la peau d'un patient, schématiquement représentée avec son épiderme 2, son derme 3 et une partie des tissus sous-cutanés 4, à l'intérieur desquels s'étend, sensiblement parallèle à la surface de la peau, une artère superficielle 5. Celle-ci présente en particulier la caractéristique d'être palpable manuellement par l'extérieur de la peau et notamment la faculté d'être momentanément obturée par suite d'une pression convenable exercée sur celle-ci depuis l'extérieur. A cet effet et de façon en elle-même connue, on peut utiliser une tête de mesure 6, dont l'extrêmité 7 venant en appui sur la peau avec interposition d'un gel de contact 7a peut, sous l'effet d'un effort d'application convenable, selon la direction de la flèche 8, venir ainsi comprimer l'artère 5 et bloquer momentanément l'écoulement sanguin dans celle-ci.

La Figure 2 illustre avec plus de détails un mode de réalisation particulier de la tête de mesure 6, comportant notamment, au voisinage de son extrêmité 7 destinée à venir en contact avec la peau 1 pour la compression de l'artère superficielle 5, une sonde DOPPLER 9, d'un type en lui-même connu dans la technique. Celle-ci comporte ainsi deux transducteurs à cristaux piézo-électriques, respectivement émetteur 10 et récepteur 11, propres à émettre une onde ultra-sonore en direction de l'artère 5 et à détecter l'onde réfléchie sur les éléments figurés du sang en circulation dans l'artère 5 et sur lesquels les ondes incidentes issues du transducteur 10 se réfléchissent pour revenir vers le transducteur 11 avec une fréquence résultante différente, fonction de la vitesse du sang en matérialisant ainsi l'effet DOPPLER bien connu.. Avantageusement, les transducteurs 10 et 11 de la sonde 9 sont orientés avec un angle , égal ou voisin de 45°, déterminé par rapport à la direction de l'axe de la tête 6, qui est le plus usuellement perpendiculaire à celle de l'artère 5, celle-ci s'étendant parallèlement à la peau, sous cette dernière. Les transducteurs 10 et 11 sont noyés et immobilisés dans la tête 6, réalisée en une résine synthétique durcie notamment.

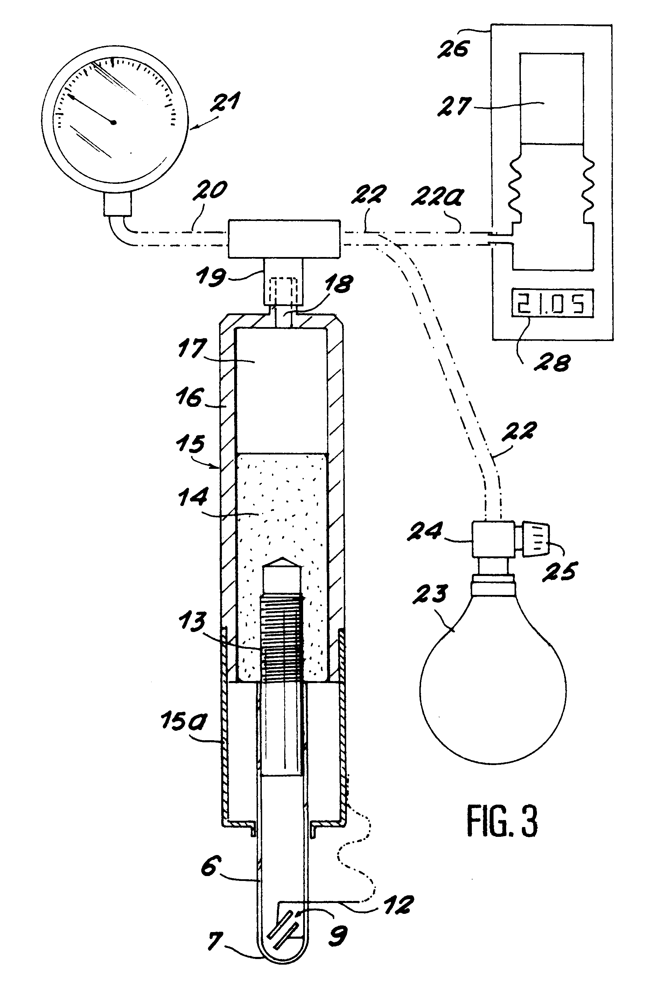

Sur la Figure 3, on retrouve la tête de mesure 6 avec sa sonde 9 noyée dans celle-ci et d'où partent des conducteurs 12, permettant de la relier à une source d'alimentation et à un ensemble de mesure extérieure, (non représentées).

La tête de mesure 6 comporte, à son extrêmité opposée à celle destinée à venir en contact avec la peau 1, une partie filetée 13, permettant de solidariser cette tête avec un piston cylindrique 14, apte à coulisser à l'intérieur d'un corps fixe 15. Celui-ci comporte une paroi fermée 16, délimitant avec le piston 14 une chambre 17 à volume variable, à l'intérieur de laquelle peut être créée une pression d'air comprimé déterminée, agissant sur le piston. A cet effet est prévu dans le fond de la chambre 17 un orifice 18, sur lequel vient s'emboîter un raccord en Té 19, permettant de relier l'intérieur de la chambre, d'une part, par une conduite 20, à un manomètre de mesure 21 et d'autre part, par une conduite 22, à une poire de gonflage manuel 23, munie d'une valve anti-retour et d'une soupape d'expansion schématiquement illustrée en 24 et commandée par une vis à molette 25. En variante et comme illustré également sur la Figure 3, le raccord 19 peut être réuni, par un prolongement 22a, à un compresseur 26, à l'intérieur duquel est montée une capsule manométrique 27. Un indicateur 28 permet en permanence de connaître la pression dans la capsule 27 et par suite à l'intérieur de la chambre 17. Le corps 15 comporte par ailleurs un capot 15a formant butée pour le piston 14, en évitant que celui-ci n'échappe au corps lorsqu'aucune pression ne s'exerce sur la tête de mesure 6.

La Figure 4 illustre un ensemble de mesure où l'appareil schématiquement illustré sur la Figure 3 est monté dans un support 29, en bout d'un bras transversal 30, permettant d'assurer son coulissement vertical sur une colonne de positionnement 31. Cette dernière est solidaire d'un plan d'appui 32, sur lequel par exemple la main 33 d'un patient peut être posée en venant s'appuyer sur un élément de réception approprié, du genre gouttière ou plaque de caoutchouc orthopédique 32a, la tête de mesure 6 étant amenée au contact de la peau du patient pour comprimer à un endroit précis préalablement déterminé, une artère superficielle de celui-ci.

Dans ce but, une fois la tête 6 amenée au contact de la peau du patient, on accroît la pression dans la chambre 17, en l'augmentant progressivement par la poire 23 ou le compresseur 26 (Figures 3), la valeur de cette pression étant lue en permanence sur le manomètre 21. Simultanément, la sonde DOPPLER 9 contenue dans la tête 6 est alimentée de telle sorte que le signal ultra-sonore qu'elle produit se réfléchisse sur les éléments figurés du sang en circulation à l'intérieur de l'artère superficielle prise en compte, jusqu'au moment où, par suite de la compression progressive de cette artère, l'écoulement s'interrompt, auquel cas la sonde DOPPLER ne produit plus aucun son. A cet instant, la pression dans la chambre 17, exactement représentative de la pression artérielle dans l'artère 5 est mesurée et lue, voire enregistrée par l'ensemble de mesure associé.

L'opération peut alors être répétée en autant d'endroits que nécessaire, en déplaçant convenablement le bras 33 du patient vis-à-vis de la tête de mesure, de telle sorte que celle-ci s'applique exactement en chacun des points souhaités. Il en va notamment ainsi lorsqu'il convient de mesurer le "pouls chinois" d'un individu, par plusieurs mesures en un nombre donné de repères anatomiques exactement connus. Les différentes valeurs de la pression, fournies par le manomètre et l'ensemble de mesure peuvent être simultanément enregistrées et conservées en mémoire, de manière à permettre toute lecture et/ou interprétation appropriée, notamment selon un programme effectuant les comparaisons souhaitées entre les différentes valeurs ainsi recueillies.

Dans une autre variante de réalisation, plus simplifiée, le boîtier du dispositif de mesure selon l'invention peut être tenu à la main par l'opérateur pour appuyer la tête de mesure 6 à l'endroit voulu du bras d'un patient.

Bien entendu et comme il va de soi, l'invention ne se limite pas aux seuls exemples de réalisation plus spécialement décrits et représentés ci-dessus ; elle en embrasse au contraire toutes les variantes. En particulier, il est clair que l'appareil selon l'invention peut s'appliquer, non seulement pour la mesure de la tension artérielle dans une artère superficielle, en particulier pour la mesure du pouls chinois d'un patient, mais également pour d'autres investigations, par exemple en cas de sténoses localisées d'une artère, en permettant en chaque point de contrôle les variations de pression instantanées, et par repérage des zones d'hyperpression en amont et d'hypopression en aval de connaître le point précis d'obstruction de l'artère.

Egalement, on pourrait envisager d'employer plusieurs sondes disposées en pont, afin de fournir une mesure simultannée en plusieurs endroits exactement localisés, avec enregistrement des mesures et mise en mémoire éventuelle de ces dernières pour toute mesure ou interprétation des résultats obtenus.

**Revendications**

1. Dispositif de mesure de la pression sanguine dans une artère superficielle, comportant une tête de mesure (6), propre à venir appuyer sur la peau (1) du patient pour comprimer l'artère superficielle (5) en provoquant, pour une pression donnée, l'arrêt de la circulation sanguine dans cette artère et, montée dans la tête, une sonde DOPPLER (9), fournissant une onde ultrasonore, réfléchie par le sang en circulation et dont l'extinction détermine le moment où se produit l'arrêt de cette circulation, caractérisé en ce que la tête de mesure est solidaire d'un piston (14) monté coulissant dans un corps fixe (15), délimitant une chambre étanche (17) dont le volume est variable selon la position de la tête par rapport au corps, et qui est remplie d'air comprimé fourni par une source de pression (23-26), la pression de l'air contenu dans la chambre étant mesurée par un ensemble de contrôle associé (21) donnant, après étalonnage, la valeur de la pression artérielle à tout instant, et en particulier à l'arrêt de la circulation sanguine dans l'artère, détecté par la sonde DOPPLER (9).

2. Dispositif de mesure selon la revendication 1, caractérisé en ce que la source de pression est constituée par un compresseur (26) ou une pompe à main (23), gonflable, munie d'une valve de non retour et d'une soupape d'échappement (24).

3. Dispositif de mesure selon l'une des revendications 1 et 2, caractérisé en ce que la tête de mesure (6) est constituée par un élément cylindrique allongé, présentant à son extrêmité opposée à celle venant comprimer l'artère superficielle, une partie filetée (13) solidaire du piston coulissant (14).

4. Dispositif de mesure selon la revendication 3, caractérisé en ce que la tête (6) est constituée par un bloc en résine durcissable dans lequel est noyée la sonde DOPPLER (9).

5. Dispositif de mesure selon l'une quelconque des revendications 1 à 4, caractérisé en ce que le corps fixe (15) comporte un capot extérieur (15a) muni d'un bord rentrant librement traversé par l'élément cylindrique de la tête de mesure et formant butée d'arrêt pour le piston coulissant (14).

6. Dispositif de mesure selon l'une quelconque des revendications 1 à 5, caractérisé en ce que l'ensemble de contrôle est muni de moyens de lecture de la fréquence des ondes ultra-sonores fournies par la sonde DOPPLER et, le cas échéant, d'un enregistreur graphique des variations de cette fréquence.

7. Dispositif de mesure selon la revendication 6, caractérisé en ce que l'ensemble de contrôle comporte également un ensemble logique, associé à un écran à cristaux liquides ou autre, comprenant une mémoire pour conserver les enregistrements des mesures et fournir une indication résultant de la comparaison de ces enregistrements.

**Claims**

1. A device for measuring blood presure in a superficial artery, comprising a measuring head (6) adapted for being pressed against a patient's skin (1) for compressing said superficial artery (5) while causing, for a given pressure, a stoppage of blood flow in this artery, and a Doppler probe (9) mounted in the measuring head (6), adapted for emitting an ultrasonic wave and for detecting the reflection thereof by the circulating blood, the extinction of the reflected wave indicating the moment of occurence of said blood flow stoppage,

 **characterized** in that the measuring head is bound up with a piston (14) slidably mounted inside a stationary barrel (15) defining a fluid-tight chamber (17) the volume of which is variable in accordance with the position of the measuring head relative to said barrel, said chamber being filled with pressurized air supplied from a pressure source (23-26), the pressure of the air contained in said chamber being measured by means of an associated control unit (21) indicating, after a calibration, the value of the arterial pressure at any time, and more particularly at the time of a blood flow stoppage in the artery being detected by the Doppler probe (9).

2. A measuring device according to Claim 1, characterized in that the pressure source is constituted by a compressor (26) or by a hand-operated inflatable pump (23) provided with a check valve and with a relief valve (24).

3. A measuring device according to one of Claims 1 and 2, characterized in that the measuring head (6) is constituted by an elongated cylindrical element presenting, on its end opposite the end which will compress the superficial artery, a threaded portion (13) bound up with the slidable piston (14).

4. A measuring device according to Claim 3, characterized in that the measuring head (6) is constituted by a block of hardenable resin in which the Doppler probe (9) is embedded.

5. A measuring device according to any one of Claims 1-4, characterized in that the stationary barrel (15) comprises an outer cap (15a) presenting an inwardly bent rim through which the cylindrical element of the measuring head is freely slidable, said rim forming a stop for the slidable piston (14).

6. A measuring device according to any one of Claims 1-5, characterized in that the control unit (21) is provided with read-out means for indicating the frequency of the ultrasonic waves from the Doppler probe and, if the case arises, with a graphic recorder for recording the variations of this frequency.

7. A measuring device according to Claim 6, characterized in that the control unit (21) further comprises a data processing unit associated with a liquid-crystal display or the like, comprising a memory for storing the recorded measurements and for giving an indication resulting from a comparison of these recordings.

**Patentansprüche**

1. Vorrichtung zur Messung des Blutdrucks in einer Oberflächenarterie mit einem Meßkopf (6), der zum Komprimieren der Oberflächenarterie (5) auf die Haut (1) des Patienten gedrückt wird, um bei einem vorgegebenen Druck den Blutfluß in dieser Oberflächenarterie zu stoppen, und mit einer im Meßkopf angeordneten Dopplersonde (9), die Ultraschallwellen aussendet, die durch das zirkulierende Blut reflektiert werden und deren Erlöschen den Moment anzeigt, in dem die Blutzirkulation aufhört, dadurch gekennzeichnet, daß der Meßkopf fest mit einem Kolben (14) verbunden ist, der in einem feststehenden Zylinder (5) verschiebbar gelagert ist, der eine geschlossene Kammer (17) abgrenzt, deren Volumen entsprechend der Position des Meßkopfes relativ zum Zylinder variabel ist und die mit komprimierter Luft aus einem Drucklufterzeuger (23-26) gefüllt ist, wobei der Luftdruck in der Kammer von einem angeschlossenen Steuergerät (21) gemessen wird, das, nach Eichung, in jedem Moment und insbesondere beim Aufhören des Blutflusses in der Arterie - angezeigt durch die Dopplersonde (9) - den Arteriendruck angibt.

2. Vorrichtung zur Messung nach Anspruch 1, dadurch gekennzeichnet, daß der Drucklufterzeuger aus einem Kompressor (26) oder einer aufblasbaren und mit einem Rückschlagventil (24) ausgestatteten Handpumpe (23) besteht.

3. Vorrichtung zur Messung nach einem der Ansprüche 1 und 2, dadurch gekennzeichnet, daß der Meßkopf (6) ein langgestecktes zylindrisches Element aufweist, das an seinem dem die Oberflächenarterie komprimierenden Ende gegenüberliegenden Ende einen Teil mit Gewinde (13) aufweist, der fest mit dem verschiebbaren Kolben (14) verbunden ist.

4. Vorrichtung zur Messung nach Anspruch 3, dadurch gekennzeichnet, daß der Meßkopf (6) einen Block aus gehärtetem Harz aufweist, in dem die Dopplersonde (9) angeordnet ist.

5. Vorrichtung zur Messung nach einem der Ansprüche 1 bis 4, dadurch gekennzeichnet, daß der feststehende Zylinder (15) eine Schutzkappe (15a) mit einem zurückspringenden Rand aufweist, der frei vom zylindrischen Element des Meßkopfes durchsetzt wird und einen Anschlag für den verschiebbaren Kolben (14) bildet.

6. Vorrichtung zur Messung nach einem der Ansprüche 1 bis 5, dadurch gekennzeichnet, daß das Steuergerät mit Mitteln zum Ablesen der von der Dopplersonde gelieferten Ultraschallfrequenz und mit einem grafischen Registrierinstrument für die Veränderungen dieser Frequenz ausgestattet ist.

7. Vorrichtung zur Messung nach Anspruch 6, dadurch gekennzeichnet, daß das Steuergerät weiterhin ein an eine Flüssigkristallanzeige oder dgl. angeschlossenes Logikgerät aufweist, das einen Speicher zum Aufzeichnen der Messergebnisse und zur Anzeige der verglichenen Meßergebnisse besitzt.

FIG. 1

FIG. 2

FIG. 4

FIG. 3